# EUROPEAN PATENT SPECIFICATION

(11) **EP 4 028 754 B1**
(45) Date of publication and mention of the grant of the patent: **03.09.2025**
(21) Application number: 20717262.8
(22) Date of filing: 28.02.2020
(51) Int. Cl.: G01N 21/51, G01N 21/53, G01N 21/64, A61K 9/127

(54) **ANALYSIS METHOD, ANALYSIS SUBSTRATE, ANALYSIS KIT, AND ANALYSIS APPARATUS**
ANALYSEVERFAHREN, ANALYSESUBSTRAT, ANALYSEKIT UND ANALYSEGERÄT
PROCÉDÉ D'ANALYSE, SUBSTRAT D'ANALYSE, KIT D'ANALYSE ET APPAREIL D'ANALYSE

(30) Priority: 10.09.2019 JP 2019164882
(43) Date of publication of application: 20.07.2022
(73) Proprietor: Kabushiki Kaisha Toshiba, Tokyo 105-0023 (JP)
(72) Inventor: KOBAYASHI, Nao, Tokyo 1050023 (JP); ISHIHARA, Mitsuko, Tokyo 1050023 (JP)
(74) Representative: Marks & Clerk LLP
(86) International application number: PCT/IB2020/051714
(87) International publication number: WO 2021/048636

(56) References cited:
- US-A1- 2013 164 400
- US-B2- 8 780 338
- SUNAMI T ET AL: "Femtoliter compartment in liposomes for in vitro selection of proteins", ANALYTICAL BIOCHEMISTRY, ACADEMIC PRESS, AMSTERDAM, NL, vol. 357, no. 1, 1 October 2006 (2006-10-01), pages 128 - 136, XP024942366, ISSN: 0003-2697, [retrieved on 20061001], DOI: 10.1016/J.AB.2006.06.040
- KAROLA VORAUER-UHL ET AL: "Determination of liposome size distribution by flow cytometry", CYTOMETRY, vol. 39, no. 2, 1 February 2000 (2000-02-01), US, pages 166 - 171, XP055698737, ISSN: 0196-4763, DOI: 10.1002/(SICI)1097-0320(20000201)39:2<166::AID-CYTO10>3.0.CO;2-M

## Description

### Cross-Reference to Related Applications

This application is based upon and claims the benefit of priority from Japanese Patent Application No. 2019-164882, filed September 10, 2019.

### Field

Embodiments described herein relate generally to an analysis method, an analysis substrate, an analysis kit, and an analysis apparatus.

### Background

In the field of disease treatment or cosmetics, as a method for delivering an objective substance (such as drugs, nucleic acids and beauty components) to a cell, there is a method for preparing a lipid particle that contains the objective substance in a lipid membrane and contacting the lipid particle with the cell.

A lipid particle is manufactured, for example, by mixing a lipid serving as a material of a lipid membrane and an objective substance in a solution. A quality of a manufactured particle group is evaluated from a fill ratio of the objective substance. A fill ratio is obtained by measuring an amount of objective substances remaining in the solution without being contained in the lipid particle and by substituting the amount into the following formula: Fill ratio = ((Total amount of objective substance input) - (Amount of objective substances remaining in solution))/(Total amount of objective substance input)

The higher the fill ratio, the higher the quality. The reason is that a higher fill ratio indicates that more objective substances are contained in a lipid particle. Furthermore, it is recommended to perform quality control to even out a fill ratio.

US 2013164400 A1 relates to a system for a process of sterilely preparing a lipid-nucleic acid nanoparticle.

SUNAMI T ET AL, ANALYTICAL BIOCHEMISTRY, ACADEMIC PRESS, 2006, vol. 357, no. 1, pages 128 - 136 relates to a strategy to estimate the internal aqueous volume of cell-size liposomes using a fluorescence-activated cell sorter.

KAROLA VORAUER-UHL ET AL, CYTOMETRY, 2000, vol. 39, no. 2, pages 166 - 171 relates to a technique using flow cytometry to characterize uni- and polydisperse suspensions.

US 8 780 338 B2 relates to an optical measuring device and method for identifying a sample.

### Brief Description of the Drawings

FIG. 1 shows a schematic view illustrating an example of a lipid particle group according to an embodiment.
FIG. 2 shows a flowchart of an example of an analysis method according to a first embodiment.
FIG. 3 shows a schematic view illustrating the example of the analysis method according to the first embodiment.
FIG. 4 shows a block diagram illustrating an example of an analysis apparatus according to the first embodiment.
FIG. 5 shows a plan view illustrating an example of an analysis substrate according to the first embodiment.
FIG. 6 shows a plan view illustrating a detector for the analysis substrate according to the first embodiment.
FIG. 7 shows a flowchart of another example of the analysis method according to the first embodiment.
FIG. 8 shows a block diagram illustrating another example of the analysis apparatus according to the first embodiment.
FIG. 9 shows a flowchart of an example of an analysis method according to a second embodiment.
FIG. 10 shows a graph showing an example of a relationship between a scattered light intensity and the number of lipid particles in the second embodiment.
FIG. 11 shows a graph showing experimental results of Example 1.
FIG. 12 shows a graph showing experimental results of Example 2.

### Detailed Description

In general, according to one embodiment, an analysis method is for determining an abundance ratio of first lipid particles containing an objective substance in a lipid particle group which contains a plurality of lipid particles. The analysis method comprising: mixing a solution containing the lipid particle group with a first substance which has lipid membrane permeability, binds to the objective substance and includes a dye that generates fluorescence, irradiating step of irradiating mixture obtained from the mixing with light that excites to generate fluorescence; measuring including a first measuring step of the number of the plurality of lipid particles in the lipid particle group by detecting scattered light obtained from the mixture by the irradiating with the light, and a second measuring step of measuring the number of the first lipid particles by detecting the fluorescence obtained from the mixture by irradiating with the light; and calculating step of calculating, based on a result obtained from the measuring, an abundance ratio of the first lipid particles by Formula (I): Abundance ratio of the first lipid particles = The number of the first lipid particles / The total number of the plurality of lipid particles in the lipid particle group.

Various embodiments will be described hereinafter with reference to the drawings. Each figure is a schematic view that promotes a better understanding about embodiments. Shapes, dimensions, ratios, and the like in each figure are different from actual ones and are designed appropriately with reference to the following description and known technology.

### First embodiment

### (Analysis method)

An analysis method of a first embodiment is to determine an abundance ratio of lipid particles containing an objective substance in a solution including a lipid particle group.

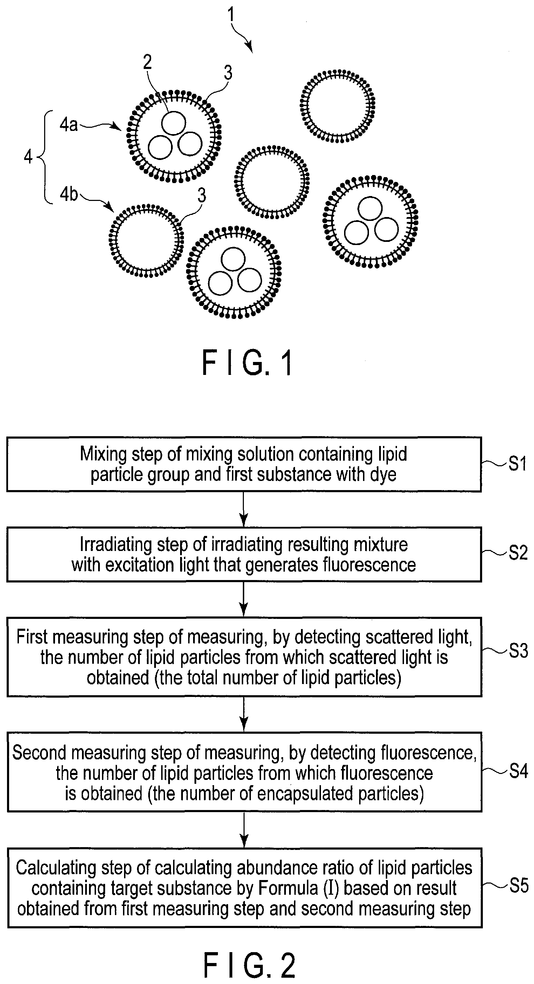

As illustrated in FIG. 1, a lipid particle group 1 includes a plurality of lipid particles 4. The lipid particles 4 may include a lipid particle 4a that contains an objective substance 2 in a lipid membrane 3 (hereinafter also referred to as "encapsulated particle(s)" or "first lipid particle(s)") and/or a lipid particle 4b that does not contain the objective substance 2 in the lipid membrane 3 (hereinafter also referred to as "non-encapsulated particle(s)" or "second lipid particle(s)"). The terms "particle" and "particles" as used herein may include both a particle (singular) and particles (plural, a plurality of particles).

The lipid membrane 3 is a substantially spherical hollow body of a lipid membrane formed by a plurality of non-covalently bonded lipid molecules.

Examples of the objective substance 2 include, but not limited to, nucleic acids, proteins, peptides, glycoproteins, low-molecular compounds, antibodies, or antigen-binding fragments. The objective substance 2 may also be, for example, drugs (such as therapeutic drugs or diagnostic drugs), cosmetic components, or genes to be delivery into cells.

Each of the plurality of lipid particles 4 included in the lipid particle group 1 desirably has a particle size, for example, less than 1 µm. A particle size of the lipid particles is measured, for example, by dynamic light scattering (DLS) or electrical sensing zone.

A solution including the lipid particle group 1 may be obtained by mixing a material of the lipid membrane 3 and the objective substance 2 (and other substances to be contained in the lipid membrane 3 as necessary) in a liquid. Alternatively, the solution may be obtained as follows: separate the lipid particle group 1 from the above liquid mixture; and add the separated lipid particle group 1 to another solvent.

The solution may be, for example, a reagent for delivery the objective substance 2 into a cell, a pharmaceutical product or a cosmetic product, which contains lipid particles. Alternatively, the solution may be a crude product a sample partially taken from a production lot in process of producing the reagent, the pharmaceutical product or the cosmetic product described above.

An analysis method according to an embodiment includes, for example, the following steps as illustrated in FIG. 2:
(S1) a mixing step of mixing the solution including the lipid particle group 1 with a first substance that has lipid membrane permeability, binds to the objective substance 2 and contains a dye which generate fluorescence;
(S2) an irradiating step of irradiating the resulting mixture with excitation light that generates fluorescence from the dye;
(S3) a first measuring step of measuring the number of lipid particles 4 causing scattered light, that is, the total number of the plurality of lipid particles by detecting scattered light;
(S4) a second measuring step of measuring the number of lipid particles 4 causing fluorescence, that is, the number of lipid particles containing the objective substance 2 (encapsulated particles 4a) by detecting fluorescence; and
(S5) a calculating step of calculating an abundance ratio of the lipid particles containing the objective substance by Formula (I): (Abundance ratio of encapsulated particles 4a = Number of encapsulated particles 4a / Total number of the plurality of lipid particles in lipid particle group), based on a result obtained from the measuring step.

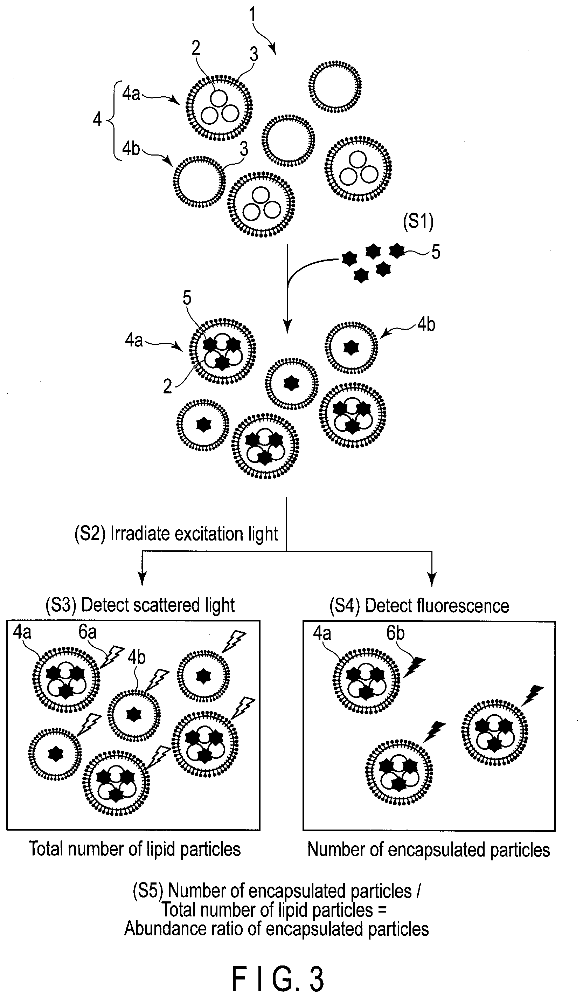

Hereinafter, each step will be described in detail with reference to FIG. 3.

A first substance 5, which is mixed with the solution including the lipid particle group 1 in the mixing step (S1), includes, for example, a dye and a binding site that binds to the objective substance 2. The first substance 5 may be a combination of two substances, that is, for example, a dye and a substance including a binding site. Alternatively, the first substance 5 may be a single substance that includes both a binding site and a dye. The first substance 5 has lipid membrane permeability as a whole.

The dye is a substance that does not generate fluorescence when being unbound to the objective substance 2 and generates fluorescence when being bound to the objective substance 2. Alternatively, as will be described in detail, the dye may be a substance that generates fluorescence with different wavelengths depending on whether the dye is bound to the objective substance 2.

Since the first substance 5 has lipid membrane permeability, the first substance 5 permeates and penetrates the lipid membrane 3 of each encapsulated particle 4a and non-encapsulated particle 4b. In each encapsulated particle 4a, the first substance 5 and the objective substance 2 are bound, which cause the dye to generate the fluorescence.

Next, in the irradiating step (S2), the mixture is irradiated with excitation light that excites the dye to generate fluorescence. The light irradiated in the irradiating step (S2) is excitation light that generates scattered light from the lipid particles 4 and excites the dye to generate fluorescence.

By the light irradiation, the light may fall on the lipid particles 4 in the lipid particle group 1 and may be scattered.

Accordingly, the total number of lipid particles is obtained by detecting the scattered light obtained from the lipid particles 4 and by counting the number of the plurality of lipid particles 4 causing scattered light 6a during the irradiating step (S2) (first measuring step (S3)).

In addition, the light irradiation excites the dye in the first substance 5. As a result, fluorescence 6b is obtained from the encapsulated particles 4a but not from the non-encapsulated particles 4b.

Accordingly, the number of encapsulated particles 4a is obtained by detecting the fluorescence 6b and by measuring the number of encapsulated lipid particles 4a causing the fluorescence 6b during the irradiating step (S2) (second measuring step (S4)). Next, in the calculating step (S5), the total number of the plurality of lipid particles 4 and the number of encapsulated particles 4a are used to calculate an abundance ratio of the encapsulated particles 4a, for example, by the following Formula (I): Abundance ratio of encapsulated particles = Number of encapsulated particles 4a / Total number of the plurality of lipid particles in lipid particle group

In this manner, an abundance ratio of the encapsulated particles 4a is determined. An abundance ratio of the encapsulated particles 4a is a new index for evaluating a quality of the lipid particle group 1.

In the related art, a fill ratio is used as an index for evaluating a quality of a lipid particle. A fill ratio is obtained by measuring an amount of residual objective substances which remains in a solution without being contained in a lipid particle. For example, in a case where the objective substance 2 is a nucleic acid, an amount of residual nucleic acid is estimated from a turbidity of a solution. In other words, this method is to evaluate a quality of a lipid particle group from estimation of a total amount of objective substances contained in lipid particles.

On the other hand, the analysis method of the embodiment enables direct measurement of the total number of lipid particles and the number of encapsulated particles 4a. The present inventors have found that an abundance ratio of the encapsulated particles 4a according to the embodiment has a high correlation with the delivery efficiency of the objective substance 2 to a cell. On the other hand, a fill ratio according to the related art has a low correlation with the delivery efficiency of the objective substance 2. This finding indicates that an abundance ratio of particles containing the objective substance 2 has a greater influence on the delivery efficiency of the objective substance 2 than an amount of the objective substances 2 contained in lipid particles. Therefore, an abundance ratio of the encapsulated particles 4a according to the embodiment may be a new index that enables more accurate evaluation of a quality of the lipid particle group 1.

For the above reasons, in producing the lipid particle group 1, when trying to uniform a quality based on a fill ratio in the related art, an abundance ratio of the encapsulated particles 4a is not always constant, which may vary the delivery efficiency to a cell. On the other hand, when a quality is controlled based on an abundance ratio of the encapsulated particles 4a by the method according to the embodiment, it is possible to produce the lipid particle group 1 with more stable introduction efficiency into a cell.

In addition, the measurement of an amount of objective substances remaining in a solution as in the related art is not always accurate, depending on compositions of a solution. However, according to the method of the embodiment, it is possible to directly count the number of encapsulated particles 4a, which makes it possible to obtain more accurate information than the method in the related art.

From the above facts, the analysis method of the embodiment provides a new index (an abundance ratio of the encapsulated particles 4a) for more accurate evaluation of a quality of the lipid particle group 1 and provides a simple method for determining the index.

Hereinafter described are details of each material and procedure used in the method of the embodiment.

A lipid molecule used as a material of the lipid membrane 3 is, for example, a main component of a biomembrane. The lipid molecule is a phospholipid cationic lipid, cholesterol, or sphingolipid, such as diacylphosphatidylcholine, diacylphosphatidylethanolamine, ceramide, sphingomyelin, dihydrosphingomyelin, kephalin, cerebroside, or the like or combinations thereof.

The lipid membrane 3 may further include other components. Examples of other components include a component that adjusts an acid dissociation constant of the lipid membrane 3, a component that controls a particle size of the lipid membrane 3, a component that increases an amount of the objective substances 2 contained, a component that facilitates fusion between the lipid membrane 3 and a cell, and/or a component that facilitates delivery of the objective substance 2 into a cell.

The lipid membrane 3 may be a lipid monomolecular membrane or a lipid bilayer membrane. Furthermore, the lipid particles may be a single-layer film or a multilayer film. The lipid membrane 3 may be particles including lipids called liposomes, vesicles or capsules.

The objective substance 2 may include one type of substance or a plurality type of substances.

The lipid particles 4 may contain a substance other than the objective substance 2 such as a drug that makes it easier for the lipid membrane 3 to contain the objective substance 2, a drug that increases an amount of the objective substances 2 contained, and/or a drug that facilitates delivery of the objective substance 2 into a cell.

A type of the first substance 5 is selected according to a type of the objective substance 2. The first substance 5 is not limited and may be any substance having a binding site that binds to the objective substance 2, including a dye that generates fluorescence by the binding, and having lipid membrane permeability.

As the first substance 5, a substance having or estimated to have the above properties may be used. Alternatively, a combination of those substances may be used. Alternatively, when it is unknown whether a candidate substance for the first substance 5 has a binding property to the objective substance 2 or has lipid membrane permeability, the first substance 5 may be determined by studying properties or permeability of the candidate substance.

A binding property to the objective substance 2 is studied by, for example, electrophoresis, column chromatography, dot blot, surface plasmon resonance measurement, or circular dichroism spectrum measurement.

Lipid membrane permeability is studied, for example, by parallel artificial membrane permeability assay in which a lipid as a material of the lipid membrane 3 is applied to an artificial membrane to assess permeability, or by a method in which a living cell is used to assess permeability into the cell. In the parallel artificial membrane permeability assay, a space in a container is separated by a porous sheet such as a membrane filter coated with lipid. One side of the space is filled with a solution including a candidate substance, and the other side of the space is filled with a solution not including a candidate molecule. The candidate substance is studied whether it transfers from one side to the other side of the space so as to study lipid membrane permeability. The lipid membrane used herein has preferably as few holes as possible, and the lipid is preferably the same as one used in the lipid membrane 3. In the method using a living cell, an animal cell such as human colon cancer-derived cell line Coco-2 is brought into contact with a candidate substance and is monitored with a microscope. When fluorescence is observed in a cell membrane, the candidate substance is determined to have lipid membrane permeability.

The binding site of the first substance 5 is not limited in type. The first substance 5 may be a substance having a binding site that binds to the objective substance 2 by any one of hydrogen bonding, ionic bonding, van der Waals forces or hydrophobic bonding.

For example, when the objective substance 2 is a nucleic acid, the first substance 5 is a nucleic acid binding stain. Examples of the nucleic acid binding stain include PicoGreen, SYBR Green, Eva Green, and AccuBlue. Alternatively, a nucleic acid having a sequence that hybridizes with a nucleic acid objective substance 2 may be used as the binding site of the first substance 5. This nucleic acid is, for example, modified to have lipid membrane permeability.

When the objective substance 2 is an antigen, the binding site of the first substance 5 may be an antigen-binding protein labeled with a fluorescent dye. The antigen-binding protein may be, for example, an antibody or a fragment thereof. The antigen-binding protein is modified and/or has a size adjusted to have lipid membrane permeability.

When the objective substance 2 is an enzyme substrate, the binding site of the first substance 5 may be a fragment having a correspondent enzyme or a binding site to the enzyme.

The binding site of the first substance 5 may be a receptor corresponding to the objective substance 2 or a fragment thereof having a binding site to the objective substance 2.

Alternatively, the above combination of the objective substance 2 and the binding site of the first substance 5 may be inverted. For example, the objective substance 2 may be an antigen-binding protein and the binding site of the first substance may be an antigen. Other combinations are also available.

The dye is not limited in type. Any dye having a wavelength in a range detected by a standard fluorescence sensor may be used.

As the dye, it is also possible to use a substance that generates fluorescence having different wavelengths depending on whether the objective substance 2 and the first substance 5 are bound. For example, it is possible to employ a dye that causes fluorescence resonance energy transfer (FRET) and varies in fluorescence wavelength when binding to the objective substance 2. In a case where the dye having a variable fluorescence wavelength is used, the excitation light irradiated which excites fluorescence after a wavelength varied by the binding is preferably used in the irradiating step (S2). Accordingly, scattered light is obtained from the plurality of lipid particles 4 in the first measuring step (S3), and fluorescence is obtained from the encapsulated particles 4a in the second measuring step (S4).

The first measuring step (S3) may be performed, for example, by capturing an image of the mixture obtained in the mixing step (S1) with a detector (for example, a camera and a microscope) that detects scattered light during the irradiating step (S2) and by counting spots where scattered light is obtained. Alternatively, the number of lipid particles may be reckoned by flowing the lipid particle group 1 through the flow channel. The method comprising; irradiating a specific region of the flow channel with the excitation light (the irradiating step (S2)); detecting scattered light in the region over time by an optical sensor; and counting the number of particles passed through the flow channel as the number of times of the scattered light is detected.

The second measuring step (S4) is performed in a similar manner to the first measuring step (S3) except that fluorescence is measured instead of scattered light. A detector to be used in this step may comprise a filter for detecting a desired fluorescence wavelength.

In the first measuring step (S3) and the second measuring step (S4), it is not always necessary to measure the number of lipid particles in the whole solution. The total number of the plurality of lipid particles 4 and the number of encapsulated particles 4a may be measured for part of the solution. Using the value as a representative value, an abundance ratio may be calculated and the resulting value may be used as an abundance ratio of the whole solution. For example, an image obtained by capturing part of the solution may also be used. In addition, an abundance ratio may be determined, for example, by calculating the total number of the plurality of lipid particles 4 and the encapsulated particles 4a per unit volume of the solution based on values of those numbers. Alternatively, an abundance ratio of part of the solution may be measured for a several times, and the average may be used as an abundance ratio of the solution.

The irradiating step (S2), the first measuring step (S3), and the second measuring step (S4) may be performed at once using, for example, an apparatus that enables measurement of both scattered light and fluorescence. An example of such an apparatus includes an apparatus using a nanoparticle tracking analysis (NTA).

### (Analysis apparatus/Analysis substrate)

The analysis method of the embodiment may be performed by an analysis apparatus.

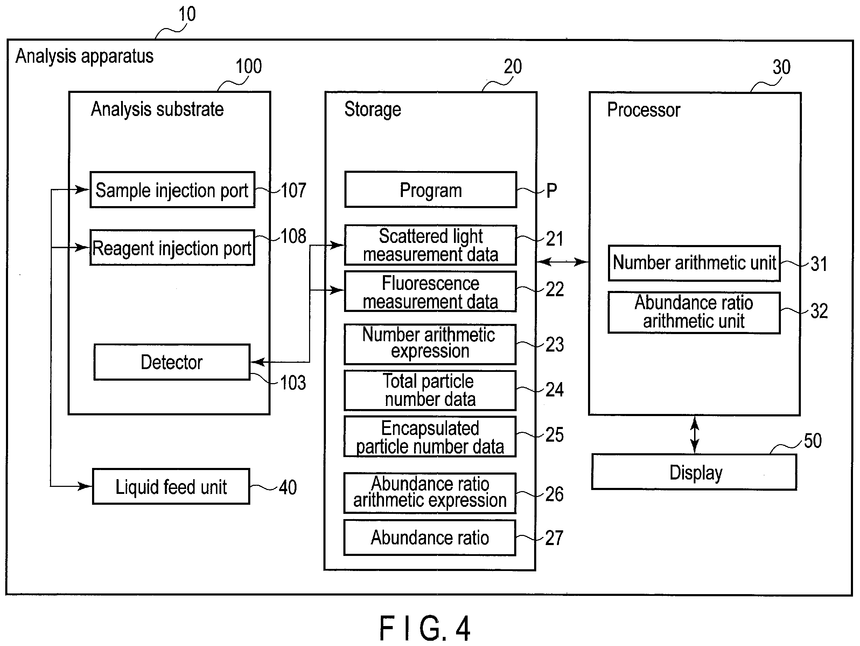

As illustrated in FIG. 4, an analysis apparatus 10 includes, for example, an analysis substrate 100, a storage 20, a processor 30, a liquid feed unit 40, and a display 50.

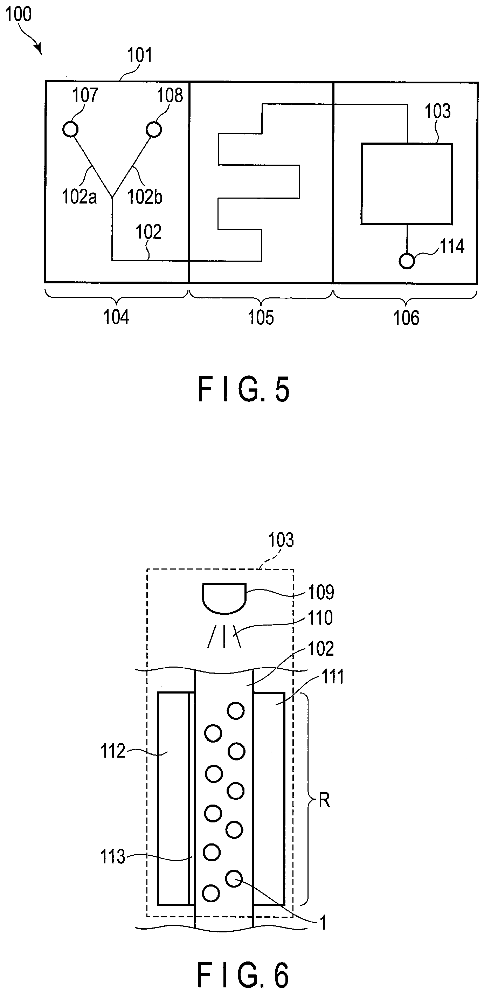

First, the analysis substrate 100 will be described with reference to FIG. 5. As illustrated in FIG. 5, the analysis substrate 100 includes a base plate 101, a flow channel 102 that is disposed on the base plate 101 and has an upstream-side end divided into two branches, and a detector 103 disposed on a downstream-side end of the flow channel 102. The analysis substrate 100 is divided into three sections according to functions. In other words, the analysis substrate 100 is provided with: a mixing unit 104 that includes the upstream-side end of the flow channel 102; a detecting unit 106 that includes the downstream-side end of the flow channel 102 and includes the detector 103; and a separating unit 105 disposed between the mixing unit 104 and the detecting unit 106.

The mixing unit 104 includes a sample injection port 107 and a reagent injection port 108. A flow channel 102a having the sample injection port 107 at the end thereof, and a flow channel 102b having the reagent injection port 108 at the end thereof are joined together and connect to the flow channel 102.

The sample injection port 107 is an opening for injecting the solution including the lipid particle group 1 into the flow channel 102. The reagent injection port 108 is an opening for injecting a solution including the first substance 5 into the flow channel 102.

The flow channel 102 leads to the separating unit 105. In the separating unit 105, the flow channel 102 is filled with a separating agent (not illustrated). The separating agent is a material that changes moving speeds of the lipid particles 4 that pass through the flow channel 102 according to a particle size. Examples of the separating agent include nanopillars, gels, nanomeshes, and porous agents. The flow channel 102 further leads to the detecting unit 106.

The detecting unit 106 includes the detector 103 disposed at a first measurement region R of the flow channel 102. As illustrated in FIG. 6, the detector 103 includes a light source 109, a scattered light detector 111, and a fluorescence detector 112. The light source 109 is configured to irradiate the lipid particle group 1 in at least the first measurement region R of the flow channel 102 with excitation light 110. The scattered light detector 111 and the fluorescence detector 112 are disposed on side surfaces of the flow channel 102 in the first measurement region R.

The scattered light detector 111 includes a scattered light sensor (not illustrated) such as a CMOS image sensor which enables detection of scattered light from the lipid particle group 1 in the flow channel 102.

The fluorescence detector 112 includes a fluorescence sensor (not illustrated) such as a CMOS image sensor which enables detection of scattered light from the lipid particle group 1 in the flow channel 102. For example, the fluorescence sensor includes a filter 113 on the side closer to the flow channel 102.

The scattered light detector 111 and the fluorescence detector 112 face each other across the flow channel 102 in FIG. 6 but are not limited to such arrangements. The scattered light detector 111 and the fluorescence detector 112 may be disposed at any positions as long as each light is detected from the lipid particle group 1 in the flow channel 102. For example, both detectors may be disposed on one side surface of the flow channel 102, and one of the detectors may be disposed close to the upstream side, and the other close to the downstream side.

As illustrated in FIG. 5, the downstream-side end of the flow channel 102 has an outlet 114.

The analysis substrate 100 does not necessarily include the separating unit 105. However, the separating unit 105 may make it possible to obtain accurate information associated with a particle size of the lipid particles 4.

The analysis substrate 100 is, for example, detachably attached to the analysis apparatus 10.

Next, other units of the analysis apparatus 10 will be described with reference to FIG. 4.

The storage 20 is electrically connected to the analysis substrate 100 and includes, for example: scattered light measurement data 21 obtained from the scattered light detector 111 (for example, an image or video obtained by capturing scattered light obtained from the lipid particle group 1, a scattered light intensity and/or the number of times the scattered light is obtained); fluorescence measurement data 22 obtained from the fluorescence detector 112 (for example, an image or video obtained by capturing fluorescence obtained from the lipid particle group 1, a fluorescence intensity and/or the number of times the fluorescence is obtained); a number arithmetic expression 23 for measuring the total number of particles based on the scattered light measurement data 21 and measuring the total number of encapsulated particles 4a based on the fluorescence measurement data 22; total particle number data 24; encapsulated particle number data 25; an abundance ratio arithmetic expression 26 for calculating an abundance ratio of the encapsulated particles 4a based on each number data; an abundance ratio 27 of the encapsulated particles 4a; and/or a program P for controlling each unit.

The processor 30 is electrically connected to the storage 20 and includes, for example, a number arithmetic unit 31 and an abundance ratio arithmetic unit 32. For example, using the number arithmetic expression 23, the number arithmetic unit 31 measures the total number of particles from the scattered light measurement data 21 and measures the number of encapsulated particles 4a from the fluorescence measurement data 22. Using the abundance ratio arithmetic expression 26, the abundance ratio arithmetic unit 32 calculates, for example, an abundance ratio of the encapsulated particles 4a from the total particle number data 24 and the encapsulated particle number data 25.

The display 50 is electrically connected to the processor 30 and the storage 20, and may include, for example, a display or a printer.

The analysis apparatus 10 may further include an input unit (not illustrated) that enables an operator to start or stop each operation of the analysis apparatus 10. The input unit includes, for example, a button and a keyboard electrically connected to the processor 30 and the storage 20, and the processor 30 may send an instruction to each unit based on an input signal. The display 50 may also include a touch-screen input unit.

The storage 20, the processor 30, the display 50, and the input unit may be configured to as a computer.

The liquid feed unit 40 includes, for example, a sample reservoir, a reagent reservoir, tubes, and pumps (not illustrated). For example, the sample reservoir is connected to the sample injection port 107 via the tube and reserves the solution including the lipid particle group 1. The reagent reservoir is connected to the reagent injection port 108 via the other tube and reserves the solution including the first substance 5. The pumps are disposed at any position in the tubes and are configured to deliver the solutions from the reservoirs to corresponding injection ports.

Hereinafter described is the analysis method using the analysis apparatus 10.

First, the solution including the lipid particle group 1 which is to be analyzed is reserved in the sample reservoir. In addition, the solution including the first substance 5 is reserved in the reagent reservoir. The analysis substrate 100 is attached to the analysis apparatus 10. The pumps of the liquid feed unit 40 are driven so that the solution including the lipid particle group 1 and the solution including the first substance 5 are delivered to the flow channel 102 from the sample reservoir and the reagent reservoir through to the sample injection port 107 and the reagent injection port 108, respectively. Accordingly, the solution including the lipid particle group 1 and the solution including the first substance 5 are mixed.

Alternatively, the solution including the lipid particle group 1 may be mixed with the first substance 5 in advance, and the resulting mixture may be injected from the sample injection port 107 and/or the reagent injection port 108. In that case, the flow channel 102 is not necessarily branched at the upstream side, and an analysis substrate with one injection port may be used.

Next, to feed the mixture to the detecting unit 106 via the separating unit 105, another solution is injected into the flow channel 102 by the liquid feed unit 40 or the solution is drawn out of the outlet 114. Lipid particles 4 that have passed through the separating unit 105 flow to the detecting unit 106 in ascending order (or descending order) of particle size.

Next, in the detector 103 of the detecting unit 106, excitation light is irradiated from the light source 109, and the scattered light detector 111 measures scattered light from the lipid particle group 1 in the flow channel 102 so as to obtain the scattered light measurement data 21. In addition, the fluorescence detector 112 measures fluorescence to obtain the fluorescence measurement data 22.

For example, the analysis substrate 100 includes pads (not illustrated) connected to each of the scattered light detector 111 and the fluorescence detector 112. The scattered light measurement data 21 and the fluorescence measurement data 22 are taken from each pad, and these pieces of data are stored in the storage 20.

Next, the number arithmetic unit 31 of the processor 30 takes the scattered light measurement data 21 and the number arithmetic expression 23 from the storage 20 and calculates the total number of lipid particles based on the number arithmetic expression 23. The total particle number data 24 is stored in the storage 20. Furthermore, the fluorescence measurement data 22 and the number arithmetic expression 23 are taken from the storage 20 and the number of encapsulated particles 4a is calculated based on the number arithmetic expression 23. The encapsulated particle number data 25 is stored in the storage 20.

Next, in the abundance ratio arithmetic unit 32 of the processor 30, the total particle number data 24, the encapsulated particle number data 25, and the abundance ratio arithmetic expression 26 are taken from the storage 20 so as to calculate the abundance ratio 27 of the encapsulated particles 4a. The abundance ratio 27 is stored in the storage 20, and the abundance ratio 27 is displayed on the display 50.

Each of the procedures may be automatically performed by the program P or may be performed manually by an operator using the input unit of the apparatus.

On completion of the analysis method, the analysis substrate 100 may be removed from the analysis apparatus 10 and discarded. When the analysis method is performed again, a new analysis substrate 100 may be used.

The analysis apparatus does not necessarily include the analysis substrate 100 and may be equipped with a sample reservoir (for example, a flow channel), a light source, a scattered light sensor and/or a fluorescence sensor.

### (Analysis kit)

According to the first embodiment, there is provided an analysis kit including the analysis substrate 100 and the first substance 5. The first substance 5 is provided, for example, in a liquid state, being included in a desired solvent. The solvent is selected according to a type of the first substance 5. Examples of the solvent include physiological saline or a buffer solution. Alternatively, the first substance 5 may be in a powder state.

### (Analysis method for evaluating a quality of a lipid particle group)

In another embodiment, the analysis method may be a method for evaluating a quality of a lipid particle group. A quality of a lipid particle group is evaluated from an index, that is, an abundance ratio of the encapsulated particles 4a obtained by Formula (I).

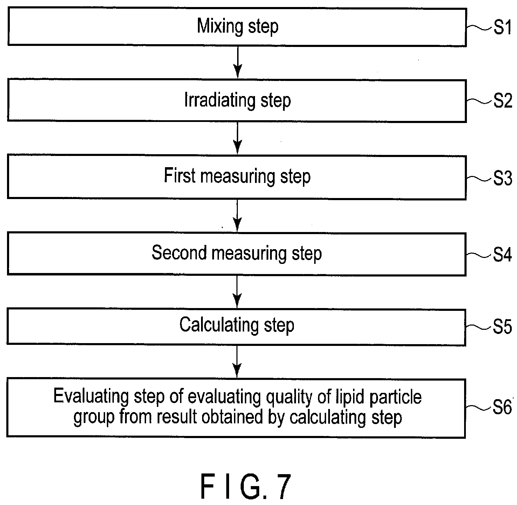

As illustrated in FIG. 7, such an analysis method includes, for example, the above steps (S1 to S5) and an evaluating step (S6). In the evaluating step (S6), a quality of a lipid particle group is evaluated from an abundance ratio as an index obtained from a result of the calculating step (S5). For example, in the evaluating step (S6), a threshold of an abundance ratio is determined in advance. When an abundance ratio is over the threshold, a quality of the lipid particle group 1 is evaluated as good. When an abundance ratio is equal to or less than the threshold, a quality of the lipid particle group 1 is evaluated as poor. The threshold is not limited and may be determined according to the use of the lipid particle group 1.

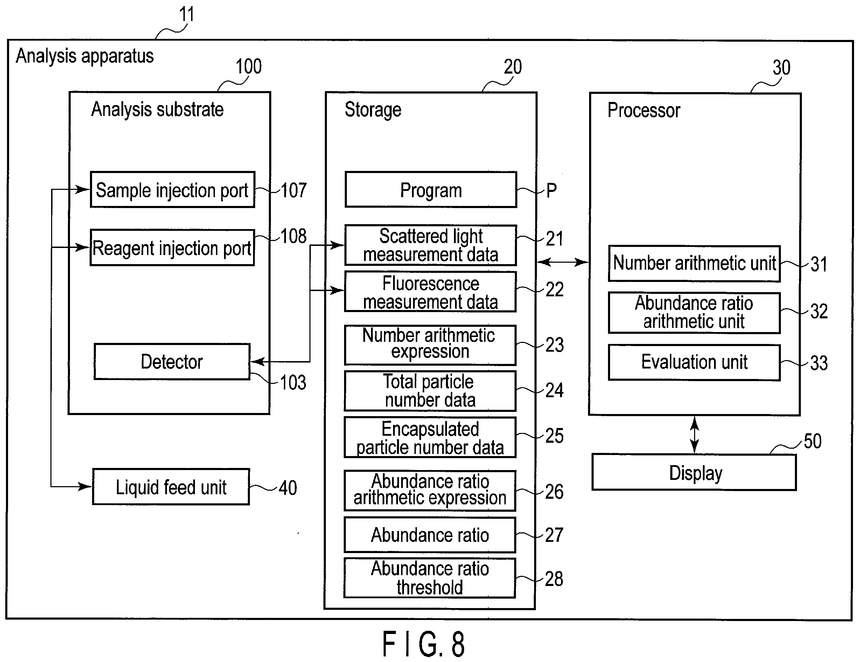

The analysis apparatus may be configured to further perform an evaluating step for the lipid particle group 1 as illustrated in FIG. 7. For example, as illustrated in FIG. 8, an analysis apparatus 11 is provided with a storage 20 that further includes an abundance ratio threshold 28, and a processor 30 that further includes an evaluation unit 33. The evaluation unit 33 compares an abundance ratio 27 with the abundance ratio threshold 28. When the abundance ratio 27 is higher than the abundance ratio threshold 28, the evaluation unit 33 displays on a display 50 that a quality of the lipid particle group 1 is good. When the abundance ratio 27 is lower than the abundance ratio threshold 28, the evaluation unit 33 may display on the display 50 that a quality of the lipid particle group 1 is poor.

In another embodiment, the analysis apparatus 10 or the analysis apparatus 11 may be, for example, attached to a device that produces the lipid particle group 1. In this case, the liquid feed unit 40 of the analysis apparatus 10 may include a sampling system that samples part of the solution including the lipid particle group 1 from a production lot and injects the solution into the sample injection port 107 of the analysis substrate 100. In addition, the processor 30 may include a changing unit that changes production parameters based on an abundance ratio or a quality evaluation result obtained by the analysis apparatus 10 or the analysis apparatus 11, and the changing unit may send instructions to the production device to change the parameters. Alternatively, in regard to a lipid particle group 1 evaluated as good based on an abundance ratio or a quality evaluation result, the processor 30 may instruct the production device to deliver the lipid particle group 1 as a final product to a line for shipping preparation.

The above analysis method and the analysis apparatus can provide a lipid particle group with a higher quality. In addition, immediately before using a lipid particle group, evaluating the lipid particle group by the analysis method or with the analysis apparatus of the embodiment enables prediction of the cell introduction efficiency according to results. Therefore, it is possible to select and use the lipid particle group 1 with a higher quality.

### Second embodiment

### (Analysis method)

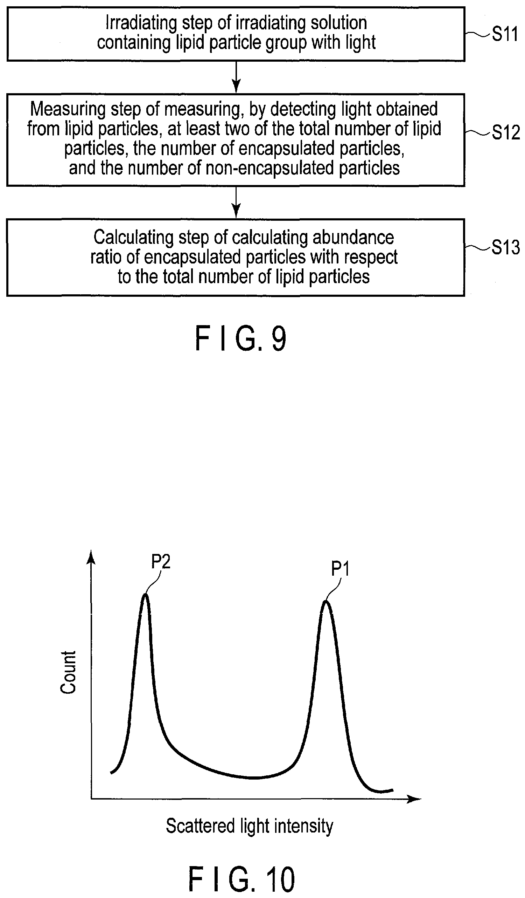

In an analysis method according to a second embodiment, an abundance ratio of encapsulated particles 4a is determined by measuring scattered light without measuring fluorescence. As illustrated in FIG. 9, the analysis method includes the following steps:
(S11) an irradiating step of irradiating a solution including a lipid particle group with light;
(S12) a measuring step of measuring at least two of the total number of the plurality of lipid particles, the number of encapsulated particles 4a, and the number of non-encapsulated particles 4b in the solution, by detecting scattered light obtained from each particle included in the lipid particle group; and
(S13) a calculating step of calculating, based on a result of the measuring step, an abundance ratio of the encapsulated particles by Formula (I) (Abundance ratio of encapsulated particles 4a = The number of encapsulated particles 4a/The total number of the plurality of lipid particles in the lipid particle group).

Hereinafter, each step will be described in detail.

The light to be irradiated in the irradiating step (S11) is not limited as long as scattered light is obtained from each particle included in a lipid particle group 1.

In the measuring step (S12), for example, a scattered light intensity obtained from each lipid particle 4 is detected. The scattered light intensity may increase with an increase in density of the lipid particles 4. Since the encapsulated particles 4a have a higher density than the non-encapsulated particles 4b, the encapsulated particles 4a may have a higher scattered light intensity.

For example, when plotting the number of lipid particles 4 with respect to the scattered light intensity, two peaks may be obtained as illustrated in FIG. 10. A peak P1 where the scattered light intensity may be large corresponds to the encapsulated particle 4a. Accordingly, the number of particles at the peak P1 is set as the number of encapsulated particles 4a. A peak P2 where the scattered light intensity may be small corresponds to the non-encapsulated particle 4b. Accordingly, the number of particles at the peak P2 is set as the number of non-encapsulated particles 4b.

When, for example, a threshold of the scattered light intensity is determined using a reference lipid particle group with a known abundance ratio of the encapsulated particles 4a, the number of particles in a range higher than the threshold is measured as the number of particles at the peak P1. On the other hand, particles in a range lower than the threshold are counted as the non-encapsulated particles 4b at the peak P2.

Regardless of the scattered light intensity, the number of lipid particles 4 causing scattered light represents the total number of lipid particles.

An abundance ratio of the encapsulated particles 4a is calculated from two of the following three values obtained as described above, that is, the total number of lipid particles, the number of encapsulated particles 4a, and the number of non-encapsulated particles 4b. Therefore, measuring at least two of the three numbers is enough.

The measuring step (S12) may be performed by, for example, nanoparticle tracking analysis (NTA). For example, analysis by NTA may be performed by a commercially available measurement instrument such as NanoSight (trade name, manufactured by Malvern Panalytical).

The calculating step (S13) may be performed in a similar manner to the calculating step (S5) of the first embodiment.

According to this method, an abundance ratio of the encapsulated particles 4a is determined without detecting fluorescence.

The analysis method of the second embodiment may also include an evaluating step similar to the evaluating step (S6).

According to another embodiment, scattered light may be detected over time in the measuring step (S12), and furthermore, a Brownian momentum of the scattered light may be measured. The Brownian momentum of the scattered light increases with an increase in particle size. Since a particle size of the encapsulated particles 4a may be larger than a particle size of the non-encapsulated particles 4b, particles with a Brownian momentum equal to or more than a threshold may be counted as the encapsulated particles 4a, and particles with a Brownian momentum equal to or less than the threshold may be counted as the non-encapsulated particles 4b.

According to another embodiment, a solution including the lipid particle group 1 and a first substance 5 may be mixed before the irradiating step (S11). Such a condition is preferable in that the scattered light intensity to be detected is further increased so that the scattered light can be detected with higher sensitivity.

It is possible to perform the analysis method according to the second embodiment with an analysis substrate and an analysis apparatus. The analysis substrate used in the analysis method may not include a fluorescence detector 112, and other configurations may be similar to those in the analysis substrate according to the first embodiment. For example, a number arithmetic unit 31 is configured to measure the number of encapsulated particles 4a and the number of non-encapsulated particles 4b from, for example, a scattered light intensity and a Brownian momentum of particles.

### [Example]

Hereinafter described are Examples in which an analysis method of the embodiment was used.

### Example 1. Measure the number of DNA-containing lipid particles

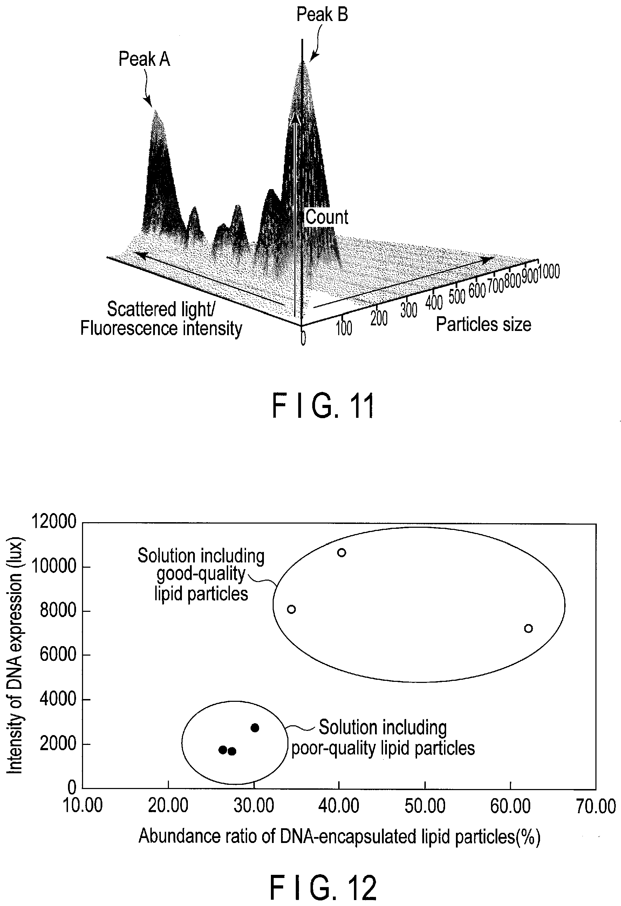

DNA and a lipid were mixed to obtain a solution including a lipid particle group. A nucleic acid binding stain (PicoGreen) having lipid membrane permeability was added to the solution. Particles were irradiated with laser light (wavelength 488 nm) by an NTA device (NanoSight, manufactured by Malvern Pnanalytical). During the irradiation, scattered light and fluorescence from each lipid particle were measured to determine the number of lipid particles that generate each light. There was prepared a scatter diagram of the particle size and the number obtained from a scattered light or fluorescence intensity and a moving speed of the Brownian motion of the particles (diffusion coefficient D) (FIG. 11).

Mainly, two peaks (A and B) were obtained in the axis of the scattered light or fluorescence intensity. Since the fluorescence intensity correlates with particle density, it is inferred that the peaks A and B represent DNA-encapsulated particles and DNA-non-encapsulated particles, respectively. Accordingly, it becomes clear that using a nucleic acid binding stain with lipid membrane permeability enables measurement of the number of DNA-encapsulated particles and DNA-non-encapsulated particles.

### Example 2. Compare method of embodiment with measurement of fill ratio

Three samples were collected from each of two lots (lot A and lot B) for producing lipid particles containing DNA including a reporter gene (the samples were referred to as samples A1, A2, A3, B1, B2, and B3). Using the samples A1 to A3 and B1 to B3, i) abundance ratio of nucleic acid-containing lipid particles, ii) fill ratio, and iii) DNA expression ratio were studied.

In regard to i), using a device similar to Example 1, an abundance ratio of DNA-encapsulated particles was calculated from Formula (I), referring the number of particles causing scattered light as the total number of lipid particles and referring the number of particles causing fluorescence as the number of DNA-encapsulated particles.

In regard to ii), an amount of residue nucleic acids remaining in a solution without being contained in a lipid particle was measured, and a fill ratio was measured from the following formula: Fill ratio = ((Amount of nucleic acids input) - (Amount of residual nucleic acids))/(Amount of nucleic acids input).

In regard to iii), each sample was brought into contact with a cell (Jurkat cell) to introduce DNA into the cell, and a value of fluorescence from the reporter gene was measured.

Table 1 and FIG. 12 show the results.

**Table 1**

| | | Number of particles (scattered light was detected) [Particles/ml] | Number of particles (fluorescence was detected) [Particles/ml] | Abundance ratio of encapsulated particles [%] | Fill ratio [%] | Fluorescence intensity (DNA expression ratio) [lux] |
|---|---|---|---|---|---|---|
| Lot A | A1 | 6.20E+12 | 1.87E+12 | 30.16 | 68.5 | 2735 |
| | A2 | 8.54E+12 | 2.26E+12 | 26.46 | 57 | 1766 |
| | A3 | 6.91E+12 | 1.90E+12 | 27.5 | 61.5 | 1659 |
| | Average | | | 28.04 | 62.3 | 2053.3 |
| Lot B | B1 | 8.19E+12 | 2.82E+12 | 34.43 | 48 | 8130 |
| | B2 | 9.01E+12 | 3.63E+12 | 40.29 | 62.45 | 10661 |
| | B3 | 8.59E+12 | 5.34E+12 | 62.17 | 69.3 | 7245 |
| | Average | | | 45.63 | 60 | 8678.7 |

As shown in Table 1, values of iii) DNA expression ratio were 2053.3 in lot A and 8678.7 in lot B, indicating that lot B had a higher value than lot A. Values of ii) fill ratio in Lot A and lot B were 62.3 and 60.0, respectively. These are substantially equivalent values and had no correlation with the result of iii). On the other hand, according to the index of i) abundance ratio of encapsulated particles, values in lot A and lot B were 28.04 and 45.63, respectively. Lot B had a higher value than lot A and had correlation with the result of iii) DNA expression ratio.

These results show that ii) fill ratio, the index in the related art, cannot accurately evaluate a quality of lipid particles (introduction efficiency of DNA). On the other hand, the results clearly show that a quality of lipid particles is more accurately evaluated from the index of i) abundance ratio of encapsulated particles in the method according to the embodiment.

This fact suggests that a quality of lipid particles (introduction efficiency of an objective substance) is more influenced by a ratio of encapsulated particles and non-encapsulated particles than an amount of nucleic acids contained in a lipid particle.

### Example 3. Study dye types

In a similar manner to in Example 1, an abundance ratio of encapsulated particles in a solution including lipid particles was determined.

In a similar manner to in Example 1, an abundance ratio of encapsulated particles was determined using a nucleic acid binding stain (Eva Green) instead of the nucleic acid binding stain (PicoGreen).

Table 2 shows the results.

**Table 2**

| | Addition amount [µl] | Number of particles (scattered light was detected) [Particles/ml] | Number of particles (fluorescence was detected) [Particles/ml] | Abundance ratio of encapsulated particles [%] |
|---|---|---|---|---|
| PicoGreen | 5 | 2.83E+12 | 8.96E+11 | 32 |
| EvaGreen | 5 | 2.84E+12 | 5.79E+11 | 20 |

When Eva Green was used, the abundance ratio of the encapsulated particles decreased compared to a case where PicoGreen was used. This is because a filter in a fluorescence measurement device used in this experiment was not suitable for detecting a fluorescence wavelength of Eva Green.

Accordingly, when using a device that enables detection of a fluorescence wavelength of Eva Green with high sensitivity, the abundance seems to be equivalent to the result in the case where PicoGreen was used.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the claims. The invention is only limited by the appended claims.

## Claims

1. An analysis method for determining an abundance ratio of first lipid particles (4a) containing an objective substance (2) in a lipid particle group (1) which contains a plurality of lipid particles (4, 4a, 4b), the analysis method comprising:
mixing a solution containing the lipid particle group with a first substance (5) which has lipid membrane permeability, binds to the objective substance and includes a dye that generates fluorescence,
irradiating a mixture obtained from the mixing with a light that excites to generate fluorescence;
measuring including a first measuring step of the number of the plurality of lipid particles in the lipid particle group by detecting scattered light obtained from the mixture by the irradiating with the light, and a second measuring step of measuring the number of the first lipid particles by detecting the fluorescence obtained from the mixture by irradiating with the light; and
calculating, based on a result obtained from the measuring, an abundance ratio of the first lipid particles by Formula (I): Abundance ratio of the first lipid particles = The number of the first lipid particles / The total number of the plurality of lipid particles in the lipid particle group.

2. The method of claim 1, wherein, in the measuring, the number of the plurality of lipid particles is measured using an intensity and/or a Brownian momentum of the scattered light as an index.

3. The method of claim 1 or 2, further comprising: evaluating a quality of the lipid particle group using the abundance ratio obtained from a result of the calculating step as an index.

4. The method of any one of claims 1 to 3, wherein the objective substance is a nucleic acid.

5. The method of any one of claims 1 to 4, wherein the plurality of lipid particles have a particle size of less than 1 µm.

6. An analysis apparatus for use in the method of any one of claims 1 to 5, the analysis apparatus comprising:
a light source configured to irradiate a mixture of a solution including a lipid particle group, which contains a plurality of lipid particles, and a first substance, with an excitation light that generates fluorescence from the dye,
wherein the first substance has lipid membrane permeability, binds to the objective substance, and includes a dye that generates a fluorescence or fluorescence having a varied wavelength,
a scattered light detector configured to detect scattered light irradiated from the mixture;
a fluorescence detector configured to detect fluorescence irradiated from the mixture; and
a processor configured to measure at least two of the total number of the plurality of lipid particles, the number of the first lipid particles, and the number of the second lipid particles in the solution based on information of light detected by the scattered light detector and the fluorescence detector, and configured to calculate an abundance ratio of the first lipid particles based on a result of the measuring in accordance with any of claims 1 to 5.

7. The analysis apparatus of claim 6, comprising
an analysis substrate,
the analysis substrate comprising:
a base plate;
a flow channel disposed on the base plate, the flow channel being configured to flow the lipid particle group from upstream to downstream;
a sample injection port disposed on the upstream side of the flow channel, the sample injection port being configured to inject at least the lipid particle group into the flow channel;
a light source configured to illuminate the lipid particle group in the flow channel with the light that excites to generate fluorescence and generates scattered light from the lipid particle group in the flow channel;
a scattered light detector disposed on the downstream side of the flow channel, the scattered light detector being configured to detect scattered light from the lipid particle group in the flow channel; and
a fluorescence detector disposed on the downstream side of the flow channel, the fluorescence detector being configured to detect fluorescence from the lipid particle group in the flow channel,
wherein the light source, the scattered light detector, and the fluorescence detector are included in the analysis substrate.

8. The analysis apparatus of claim 7, wherein the analysis substrate further comprises: a reagent injection port disposed on the upstream side of the flow channel, the reagent injection port being configured to inject the first substance into the flow channel.

9. The analysis apparatus of claim 7 or 8, wherein the analysis substrate further comprises: a separating unit disposed between the sample injection port and the scattered light detector and the fluorescence detector,
wherein the separating unit changes order in which the lipid particles flow in order of particle size.

10. An evaluating method for evaluating a quality of a lipid particle group which contains a plurality of lipid particles,
wherein the method comprising:
evaluating the quality of the lipid particle group by using, as an index, an abundance ratio of a first lipid particles containing the objective substance obtained by Formula (I): Abundance ratio of the first lipid particles = The number of the first lipid particles /The total number of the plurality of lipid particles in the lipid particle group,
wherein the abundance ratio of the first lipid particles is measured by the method of any one of claims 1 to 5.

## Patentansprüche

1. Analyseverfahren zum Bestimmen eines Häufigkeitsverhältnisses von ersten Lipidpartikeln (4a), die eine Zielsubstanz (2) enthalten, in einer Lipidpartikelgruppe (1), die eine Vielzahl von Lipidpartikeln (4, 4a, 4b) enthält, wobei das Analyseverfahren Folgendes umfasst:
Mischen einer Lösung, welche die Lipidpartikelgruppe enthält, mit einer ersten Substanz (5), die Lipidmembranpermeabilität aufweist, an die Zielsubstanz bindet und einen Farbstoff einschließt, der Fluoreszenz erzeugt,
Bestrahlen einer durch das Mischen erlangten Mischung mit einem Licht, das zur Erzeugung von Fluoreszenz anregt;
Messen, einschließlich eines ersten Messschritts der Anzahl der Vielzahl von Lipidpartikeln in der Lipidpartikelgruppe durch Erfassen von Streulicht, das aus der Mischung durch das Bestrahlen mit dem Licht erlangt wird, und eines zweiten Messschritts eines Messens der Anzahl der ersten Lipidpartikel durch Erfassen der Fluoreszenz, die aus der Mischung durch Bestrahlen mit dem Licht erlangt wird; und
Berechnen eines Häufigkeitsverhältnisses der ersten Lipidpartikel auf der Grundlage eines durch das Messen erlangten Ergebnisses durch Formel (I): Häufigkeitsverhältnis der ersten Lipidpartikel = die Anzahl der ersten Lipidpartikel / die Gesamtanzahl der Vielzahl von Lipidpartikeln in der Lipidpartikelgruppe.

2. Verfahren nach Anspruch 1, wobei bei dem Messen die Anzahl der Vielzahl von Lipidpartikeln unter Verwendung einer Intensität und/oder eines Brown'schen Impulses des Streulichts als einem Index gemessen wird.

3. Verfahren nach Anspruch 1 oder 2, ferner Folgendes umfassend: Bewerten einer Qualität der Lipidpartikelgruppe unter Verwendung des Häufigkeitsverhältnisses, das aus einem Ergebnis des Berechnungsschritts erlangt wird, als einem Index.

4. Verfahren nach einem der Ansprüche 1 bis 3, wobei die Zielsubstanz eine Nukleinsäure ist.

5. Verfahren nach einem der Ansprüche 1 bis 4, wobei die Vielzahl von Lipidpartikeln eine Partikelgröße von weniger als 1 µm aufweist.

6. Analysevorrichtung zur Verwendung in dem Verfahren nach einem der Ansprüche 1 bis 5, wobei die Analysevorrichtung Folgendes umfasst:
eine Lichtquelle, die dafür konfiguriert ist, eine Mischung aus einer Lösung, die eine Lipidpartikelgruppe einschließt, welche eine Vielzahl von Lipidpartikeln enthält, und einer ersten Substanz mit einem Anregungslicht zu bestrahlen, das Fluoreszenz aus dem Farbstoff erzeugt,
wobei die erste Substanz Lipidmembranpermeabilität aufweist, an die Zielsubstanz bindet und einen Farbstoff einschließt, der eine Fluoreszenz oder eine Fluoreszenz mit einer variierten Wellenlänge erzeugt,
einen Streulichtdetektor, der dafür konfiguriert ist, Streulicht zu erfassen, das von der Mischung ausgestrahlt wird;
einen Fluoreszenzdetektor, der dafür konfiguriert ist, Fluoreszenz zu erfassen, die von der Mischung ausgestrahlt wird; und
einen Prozessor, der dafür konfiguriert ist, auf der Grundlage von Informationen über Licht, das durch den Streulichtdetektor und den Fluoreszenzdetektor erfasst wird, mindestens zwei von der Gesamtanzahl der Vielzahl von Lipidpartikeln, der Anzahl der ersten Lipidpartikel und der Anzahl der zweiten Lipidpartikel in der Lösung zu messen, und dafür konfiguriert ist, ein Häufigkeitsverhältnis der ersten Lipidpartikel auf der Grundlage eines Ergebnisses des Messens nach einem der Ansprüche 1 bis 5 zu berechnen.

7. Analysevorrichtung nach Anspruch 6, umfassend ein Analysesubstrat, wobei das Analysesubstrat Folgendes umfasst:
eine Grundplatte;
einen Strömungskanal, der auf der Grundplatte angeordnet ist, wobei der Strömungskanal dafür konfiguriert ist, die Lipidpartikelgruppe von stromaufwärts nach stromabwärts strömen zu lassen;
einen Probeninjektionsanschluss, der auf der stromaufwärts gelegenen Seite des Strömungskanals angeordnet ist, wobei der Probeninjektionsanschluss dafür konfiguriert ist, mindestens die Lipidpartikelgruppe in den Strömungskanal zu injizieren;
eine Lichtquelle, die dafür konfiguriert ist, die Lipidpartikelgruppe in dem Strömungskanal mit dem Licht zu beleuchten, das zur Erzeugung von Fluoreszenz anregt und Streulicht von der Lipidpartikelgruppe im Strömungskanal erzeugt;
einen Streulichtdetektor, der auf der stromabwärts gelegenen Seite des Strömungskanals angeordnet ist, wobei der Streulichtdetektor dafür konfiguriert ist, Streulicht von der Lipidpartikelgruppe in dem Strömungskanal zu erfassen; und
einen Fluoreszenzdetektor, der auf der stromabwärts gelegenen Seite des Strömungskanals angeordnet ist, wobei der Fluoreszenzdetektor dafür konfiguriert ist, Fluoreszenz von der Lipidpartikelgruppe in dem Strömungskanal zu erfassen,
wobei die Lichtquelle, der Streulichtdetektor und der Fluoreszenzdetektor in das Analysesubstrat eingebunden sind.

8. Analysevorrichtung nach Anspruch 7, wobei das Analysesubstrat ferner Folgendes umfasst: einen Reagenzinjektionsanschluss, der auf der stromaufwärts gelegenen Seite des Strömungskanals angeordnet ist, wobei der Reagenzinjektionsanschluss dafür konfiguriert ist, die erste Substanz in den Strömungskanal zu injizieren.

9. Analysevorrichtung nach Anspruch 7 oder 8, wobei das Analysesubstrat ferner Folgendes umfasst: eine Trenneinheit, die zwischen dem Probeninjektionsanschluss und dem Streulichtdetektor und dem Fluoreszenzdetektor angeordnet ist,
wobei die Trenneinheit die Reihenfolge, in der die Lipidpartikel strömen, in der Reihenfolge der Partikelgröße ändert.

10. Bewertungsverfahren zur Bewertung einer Qualität einer Lipidpartikelgruppe, die eine Vielzahl von Lipidpartikeln enthält,
wobei das Verfahren Folgendes umfasst:
Bewerten der Qualität der Lipidpartikelgruppe, indem ein Häufigkeitsverhältnis von ersten Lipidpartikeln, welche die Zielsubstanz enthalten, als ein Index verwendet wird, das durch Formel (I) erlangt wird: Häufigkeitsverhältnis der ersten Lipidpartikel = die Anzahl der ersten Lipidpartikel / die Gesamtanzahl der Vielzahl von Lipidpartikeln in der Lipidpartikelgruppe,
wobei das Häufigkeitsverhältnis der ersten Lipidpartikel durch das Verfahren nach einem der Ansprüche 1 bis 5 gemessen wird.

## Revendications

1. Procédé d'analyse pour déterminer un rapport d'abondance de premières particules lipidiques (4a) contenant une substance objet (2) dans un groupe de particules lipidiques (1) qui contient une pluralité de particules lipidiques (4, 4a, 4b), le procédé d'analyse comprenant :
le mélange d'une solution contenant le groupe de particules lipidiques avec une première substance (5) qui présente une perméabilité de la membrane lipidique, qui se lie à la substance objet et qui inclut un colorant qui génère de la fluorescence,
l'irradiation d'un mélange obtenu à partir du mélange à l'aide d'une lumière qui provoque une excitation pour générer de la fluorescence ;
la mesure incluant une première étape de mesure du nombre de la pluralité de particules lipidiques dans le groupe de particules lipidiques en détectant une lumière diffusée obtenue à partir du mélange par l'irradiation à l'aide de la lumière, et une seconde étape de mesure consistant à mesurer le nombre des premières particules lipidiques en détectant la fluorescence obtenue à partir du mélange par l'irradiation à l'aide de la lumière ; et
le calcul, sur la base d'un résultat obtenu à partir de la mesure, d'un rapport d'abondance des premières particules lipidiques au moyen de la Formule (I) : rapport d'abondance des premières particules lipidiques = nombre des premières particules lipidiques/nombre total de la pluralité de particules lipidiques dans le groupe de particules lipidiques.

2. Procédé selon la revendication 1, dans lequel, lors de la mesure, le nombre de la pluralité de particules lipidiques est mesuré en utilisant une intensité et/ou un moment brownien de la lumière diffusée en tant qu'index.

3. Procédé selon la revendication 1 ou 2, comprenant en outre : l'évaluation d'une qualité du groupe de particules lipidiques en utilisant le rapport d'abondance obtenu à partir d'un résultat de l'étape de calcul en tant qu'index.

4. Procédé selon l'une quelconque des revendications 1 à 3, dans lequel la substance objet est un acide nucléique.

5. Procédé selon l'une quelconque des revendications 1 à 4, dans lequel la pluralité de particules lipidiques présentent une taille de particules inférieure à 1 µm.

6. Appareil d'analyse pour une utilisation dans le procédé selon l'une quelconque des revendications 1 à 5, l'appareil d'analyse comprenant :
une source de lumière configurée pour irradier un mélange d'une solution incluant un groupe de particules lipidiques, lequel contient une pluralité de particules lipidiques, et une première substance, à l'aide d'une lumière d'excitation qui génère de la fluorescence à partir du colorant,
dans lequel la première substance présente une perméabilité de la membrane lipidique, se lie à la substance objet et inclut un colorant qui génère une fluorescence ou une fluorescence présentant une longueur d'onde diversifiée ;
un détecteur de lumière diffusée configuré pour détecter une lumière diffusée irradiée depuis le mélange ; et
un détecteur de fluorescence configuré pour détecter une fluorescence irradiée depuis le mélange ; et
un processeur configuré pour mesurer au moins deux nombres parmi le nombre total de la pluralité de particules lipidiques, le nombre des premières particules lipidiques et le nombre des secondes particules lipidiques dans la solution sur la base d'une information de lumière détectée par le détecteur de lumière diffusée et le détecteur de fluorescence, et configuré pour calculer un rapport d'abondance des premières particules lipidiques sur la base d'un résultat de la mesure selon l'une quelconque des revendications 1 à 5.

7. Appareil d'analyse selon la revendication 6, comprenant un substrat d'analyse, le substrat d'analyse comprenant :
une plaque de base ;
un canal d'écoulement disposé sur la plaque de base, le canal d'écoulement étant configuré pour faire s'écouler le groupe de particules lipidiques de l'amont vers l'aval ;
un orifice d'injection d'échantillon disposé sur le côté amont du canal d'écoulement, l'orifice d'injection d'échantillon étant configuré pour injecter au moins le groupe de particules lipidiques à l'intérieur du canal d'écoulement ;
une source de lumière configurée pour éclairer le groupe de particules lipidiques dans le canal d'écoulement à l'aide de la lumière qui provoque une excitation pour générer de la fluorescence et génère une lumière diffusée à partir du groupe de particules lipidiques dans le canal d'écoulement ;
un détecteur de lumière diffusée disposé sur le côté aval du canal d'écoulement, le détecteur de lumière diffusée étant configuré pour détecter une lumière diffusée à partir du groupe de particules lipidiques dans le canal d'écoulement ; et
un détecteur de fluorescence disposé sur le côté aval du canal d'écoulement, le détecteur de fluorescence étant configuré pour détecter une fluorescence à partir du groupe de particules lipidiques dans le canal d'écoulement,
dans lequel la source de lumière, le détecteur de lumière diffusée et le détecteur de fluorescence sont inclus dans le substrat d'analyse.

8. Appareil d'analyse selon la revendication 7, dans lequel le substrat d'analyse comprend en outre : un orifice d'injection de réactif disposé sur le côté amont du canal d'écoulement, l'orifice d'injection de réactif étant configuré pour injecter la première substance à l'intérieur du canal d'écoulement.

9. Appareil d'analyse selon la revendication 7 ou 8, dans lequel le substrat d'analyse comprend en outre : une unité de séparation disposée entre l'orifice d'injection d'échantillon et le détecteur de lumière diffusée et le détecteur de fluorescence,
dans lequel l'unité de séparation modifie l'ordre dans lequel les particules lipidiques s'écoulent selon l'ordre des tailles de particules.

10. Procédé d'évaluation pour évaluer une qualité d'un groupe de particules lipidiques qui contient une pluralité de particules lipidiques,
dans lequel le procédé comprend
l'évaluation de la qualité du groupe de particules lipidiques en utilisant, en tant qu'index, un rapport d'abondance de premières particules lipidiques contenant la substance objet obtenue par la Formule (I) : rapport d'abondance des premières particules lipidiques = nombre des premières particules lipidiques/nombre total de la pluralité de particules lipidiques dans le groupe de particules lipidiques,
dans lequel le rapport d'abondance des premières particules lipidiques est mesuré par le procédé selon l'une quelconque des revendications 1 à 5.
